# EUROPEAN PATENT SPECIFICATION

(11) **EP 2 718 263 B1**
(45) Date of publication and mention of the grant of the patent: **20.04.2016**
(21) Application number: 11827762.3
(22) Date of filing: 28.09.2011
(51) Int. Cl.: C07D 401/12

(54) **METHOD FOR PREPARING HIGHLY PURIFIED BENIDIPINE HYDROCHLORIDE**
VERFAHREN ZUR HERSTELLUNG VON REINEM BENIDIPIN-HYDROCHLORID
PROCEDE DE PREPARATION DE CHLORHYDRATE DE BENIDIPINE PUR

(30) Priority: 18.04.2011 CN 201110096840
(43) Date of publication of application: 16.04.2014
(73) Proprietor: Hefei Beini Medical Technology Company, Ltd, Anhui 230601 (CN)
(72) Inventor: ZHANG, Zhaoyong, Anhui 230601 (CN)
(74) Representative: Awapatent A/S
(86) International application number: PCT/CN2011/080293
(87) International publication number: WO 2012/142815

(56) References cited:
- EP-A1- 0 063 365
- EP-A2- 0 106 275
- JP-A- 61 158 962
- JP-A- 2005 289 894

## Description

### Field of the invention

The present invention belongs to medicine field and relates to a method of preparing highly purified benidipine hydrochloride by ultrasonic technology. Specifically, the method of the present invention comprises: 1) during the pre-treatment stage, including acylchloridizing the main ring of dihydropyridine and then linking the side chain to synthesize directly benidipine hydrochloride, ultrasound technology is introduced, and the reactant material is dispersed and dissolved by ultrasound, and/or 2)during the post-treatment stage, ultrasonic technology is introduced and crude crystal (or product obtained by heating to dryness) of benidipine hydrochloride obtained via the method described above or other possible synthetic routes is dissolved once more and then highly purified benidipine hydrochloride is obtained by ultrasound.

### Background of the invention

Benidipine hydrochloride, whose chemical name is (±)-(*R**)-1,4-dihydro-2,6-dimethyl-4-(meta-nitrophenyl)-3,5-pyridinedica rbolate methyl ester [(R*)-1-benzyl-3-piperidine alcohol ester], belongs to dihydropyridine receptor antagonist. It can bind to dihydropyridine receptors at the binding site with high affinity and high specificity, and shows a strong inhibitory effect on Ca²⁺ channel. Benidipine not only has an inhibitory effect on muscular (L-type) Ca²⁺ channel, but also has an inhibitory effect on voltage-dependent N- and T-type Ca²⁺ channels. It is, up to now, the only calcium antagonist that can inhibit all the three Ca²⁺ channels mentioned above. Furthermore, benidipine has highly affinity with cell membrane, has vascular selectivity and renal protection effect. Therefore, it is an ideal, safe and effective agent for the treatment of hypertension and renal parenchymal hypertension and angina.

There are two chiral atoms in the molecule of benidipine hydrochloride, which locate on site 4 of the dihydropyridine ring and site 3' of the side chain piperidine ring. Accordingly, benidipine hydrochloride has 4 optical isomers: (S)-(S)-(+)-α, (R)-(R)-(-)-α, (R)-(S)-(+)-β and (S)-(R)-(-)-β, and the active ingredients for drug are the mixture of (S)-(S)-(+)-α and (R)-(R)-(-)-α. Therefore, it is necessary to separate α and β isomers during the post-treatment stage of benidipine hydrochloride preparation.

Based on the order of synthesis of dihydropyridine main ring, there mainly are two groups of total 5 synthesis routes of benidipine hydrochloride. Among them, there are two routes which involve synthesis of the main ring first: 1) acylchloridizing the main ring of dihydropyridine and then linking the side chain to synthesize directly benidipine hydrochloride; 2) After acylchloridizing the main ring of dihydropyridine, 3-piperidinol and then benzyl is added. The routes involve the synthesis of the main ring later includes the following; 1) synthesizing the main ring via β-aminocrotonate; 2) synthesizing the main ring via acetylacetate ester; 3) the 'one-pot' method involving 3-nitrobenzaldehyde, β-aminocrotonate and acetylaceate ester.

Several synthetic routes of benidipine hydrochloride and its analogues have been disclosed in EP0063365A1, EP0161877A2, JP57-171968A, EP0106275A2, etc. Among them, EP0106275A2 gave a summary of the synthetic pathways of benidipine hydrochloride. In all of the above references, it was mentioned to separate the benidipine hydrochloride prepared through column chromatography and spit it into its α and β isomers, thus obtain the therapeutically active (+)-α- benidipine hydrochloride.

In order to obtain a highly purified benidipine hydrochloride meeting pharmaceutical use, it is necessary to perform multiple recrystallization with acetone and/or ethanol. Moreover, the crystallization condition is relatively strict since it should be performed below freezing point or even below -20°C. Furthermore, the crystallization process usually need a relatively long time (more than 24 hours).

JP2007-8819A thus disclosed a method for preparing highly purified benidipine hydrochloride meeting pharmaceutical use by first preparing the monohydrate of benidipine hydrochloride.

All of the methods mentioned above involve multiple steps, and even require column chromatography to effectively separate the isomers. These methods are not only time and labor consuming, but also need great amount of organic solvents, which will bring about negative impacts on both the society wealth and environmental protection.

### Summary of the invention

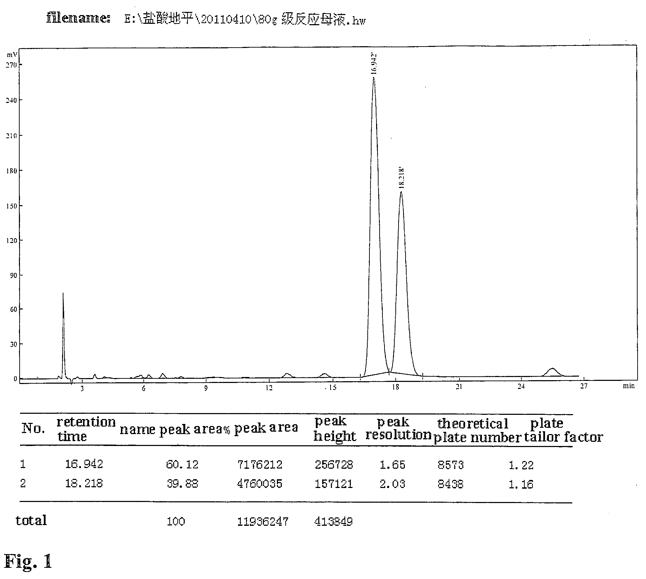

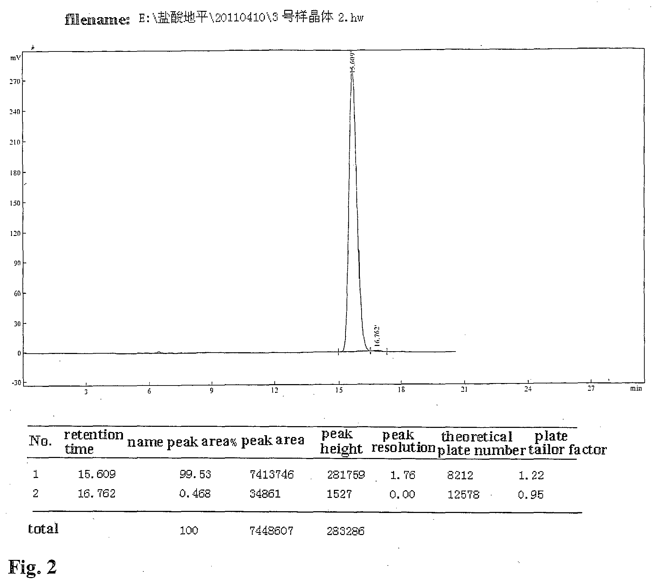

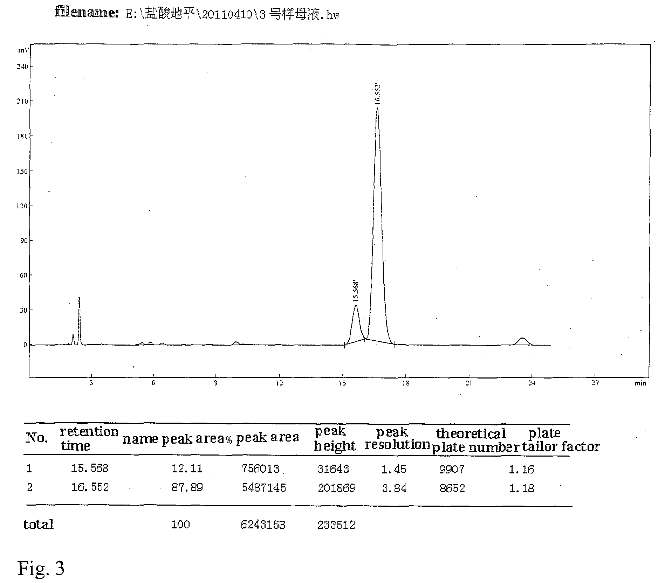

The present invention mainly uses the direct synthesis method of benidipine hydrochloride including acylchloridizing main ring of dihydropyridine and then linking the side chain. 1) During the pre-treatment stage for preparing benidipine hydrochloride, ultrasound technology is introduced, and the reactant material is dispersed and dissolved by untrasound, and thus the reactant disperses evenly into the reaction medium to render the reaction perform stably and rapidly. Therefore, the reaction rate is enhanced. 2) During the post-treatment stage, the benidipine hydrochloride, which is obtained by the method described above or by other possible synthesis routes, is formed into crude crystal of benidipine hydrochloride by heating reaction solvent to dryness or other feasible methods. Then after the crude crystal of benidipine hydrochloride or the products obtained from heating to dryness are dissolved a suitable solvent, ultrasound technology is introduced. By ultrasound crystallization, unexpected effective separation of benidipine hydrochloride isomers may be achieved. The purity of benidipine hydrochloride α isomers obtained is above 98.6% (HPLC area normalization method), the crystallization duration is shortened from 24 hours to 30 minutes, and the yield is also increased greatly. Figure 1 is a liquid chromatography of benidipine hydrochloride crude crystal or product obtained by heating to dryness using HPLC area normalization method; Figure 2 is a liquid chromatography of ultrasound crystal obtained using HPLC area normalization method; and Figure 3 is a liquid chromatography of mother liquor after crystallization by ultrasound using HPLC area normalization method.

The present invention not only avoids the separation process by column chromatography, and simplifies the process to make operation easier, and reduces use and discharge of organic solvents, but also allows saving of human and material resources, and time and cost, and is in favor of environmental protection.

During the pre- and post-treatment stages of benidipine hydrochloride synthesis, suitable temperature for ultrasound crystallization is -78°C∼100 °C, preferably -5°C to 30°C. Suitable ultrasound frequency is 20 kHz∼ 500 kHz, preferably 20 kHz∼100 kHz. Suitable ultrasound power is 1 mW∼5000 W, preferably 1 W∼500 W. The duration of the ultrasound is 1 minute to 24 hours, preferably 3 minutes to 120 minutes. Suitable solvent for preparing benidipine hydrochloride under ultrasound is lower-carbon-number ketone, lower-carbon-number alcohol, lower-carbon-number ether, lower-carbon-number acid, lower-carbon-number ester, dichloromethane, chloroform, acetic anhydride or commonly used small molecule solvents. Preferred solvents are selected from the group consisting of acetone, ethanol, methanol, N,N-dimethylformamide (DMF), acetonitrile, diethyl ether, dichloromethane and water.

The preparation procedure of benidipine hydrochloride in the present invention are as follows:
(1) Under ultrasound condition, dihydropyridine main ring [chemical name: 2,6-dimethyl-4-(3-nitrophenyl)-1,4-dihydropyridine-3,5-dicarboxylic acid monomethyl ester] is dispersed evenly in appropriate amount of mixed solution of N,N-dimethylformamide (DMF) and dichloromethane. The resultant suspension is cooled in ice-bath condition, and thionyl chloride is added. The mixture is stirred until the solution becomes clear and the main ring, i.e., monochloro acylate [monochloro acylated 2,6-dimethyl-4-(3-nitrophenyl)-1,4-dihydropyridine-3,5-dicarboxylic acid monomethyl ester] is obtained.
(2) Under or not under ultrasound conditions, add reactive amount of pyridine (alcohol) side chain [1-benzyl-3-hydroxypiperidine], and allow reaction for a certain period of time.
(3) The reaction solution is washed with water and saturated saline for several times. Solvent is recycled to obtain crude crystal of benidipine hydrochloride (containing α and β isomers, and other related substances).
(4) The crude crystal of benidipine hydrochloride (obtained via the reactions described above or via other suitable synthesis routes) is dissolved in suitable single or mixed solvent, and ultrasound is applied for a certain time at a suitable power. After one or more times of crystallization, α-benidipine hydrochloride is obtained directly as light yellow powdery crystal, with a purity of over 98.6% (HPLC area normalization method).

### Brief description of the drawings

Fig. 1 is a liquid chromatography of benidipine hydrochloride crude crystal or product obtained by heating to dryness using HPLC area normalization method;
Fig. 2 is a liquid chromatography of ultrasound crystal obtained using HPLC area normalization method;
Fig. 3 is a liquid chromatography of mother liquor after crystallization by ultrasound using HPLC area normalization method.

### Detailed description of the invention

The present invention will be described in detail in combination with the following Examples.

### Example 1

Under ultrasound, 10 g of dihydropyridine main ring , i.e.,[2,6-dimethyl -4-(3-nitrophenyl)-1,4-dihydropyridine-3,5-dicarboxylic acid monomethyl ester] was placed into 200 mL reaction flask, and 14 mL N,N-dimethylformamide (DMF) and 56 mL dichloromethane was added. To the resultant homogeneous suspension was added 2.4 mL of thionyl chloride under ice-bath, then the mixture was stirred for 1 hour to obtain a clear solution.

Then, 6.3 g of pyridine (alcohol) side chain, i.e., [1-benzyl-3 -hydroxypiperidine] was added and stirred for 2.5 hours under ice-bath.

The reaction solution was washed with 40 mL water (× 4) and 40 mL saturated saline solution (× 1). The dichloromethane solution was dried for two hours by adding 4 g of anhydrous sodium sulfate. Then, sodium sulfate solid was removed by filtering, and dichloromethane was recycled under reduced pressure to obtain a yellow to red crude crystal (herein after referred to as crude crystal of benidipine hydrochloride).

The crude crystal mentioned above was dissolved in 100 mL acetone, and ultrasounded at 150 W and 40 MHz for 7 minutes, filtered under reduced pressure and dried to obtain 5.9 g of crystal as yellow powder (yield 36.2%).

### Example 2

3.0 g of 3-nitrobenzaldehyde, 2.6 g of β-aminocrotonate and 2.6 g of ethyl acetoacetate were dissolved in a mixed solvent of 10 mL DMF and 50 mL dichloromethane. The reaction mixture was stirred for 3 h at 50°C, washed with water, dried. The solvent was recycled to obtain a yellow to red crude crystal of benidipine hydrochloride.

### Example 3

10g of benidipine hydrochloride crude crystal was dissolved in 60 mL acetone. Ultrasonification was performed for 8 minutes at 200 W and 80 MHz. The reaction mixture was filtered under reduced pressure. Then the crystal above was dissolved in 5 ml ethanol by heating under refluxing. 40 mL acetone was added under untrasonification at 150 W and 40 MHz and ultrasonification was continued for 8 minutes. The mixture was filtered under reduced pressure and dried to obtain 3.2 g of crystal as light yellow powder.

### Example 4

10g of benidipine hydrochloride crude crystal was dissolved in 100 mL acetone + 1 mL absolute ethanol. Ultrasonification was performed for 10 minutes at 150 W and 40 MHz. The mixture was filtered under reduced pressure and dried to obtain light yellow powdery crystal.

### Example 5

10g of benidipine hydrochloride crude crystal was dissolved in 100 mL acetone + 5 mL water. Ultrasonification was performed for 17 minutes at 150 W and 40 MHz. The mixture was filtered under reduced pressure and dried to obtain light yellow powdery crystal.

### Example 6

10g of benidipine hydrochloride crude crystal was dissolved in 100 mL isopropanol. 7 mL acetonitrile was added under ultrasonification at 150 W and 40 MHz and untrasonification was continued for 10 minutes. The mixture was filtered under reduced pressure and dried to obtain light yellow powdery crystal.

### Example 7

10g of benidipine hydrochloride crude crystal was dissolved in 10 mL DMF. 10 mL water was added under ultrasonification at 250 W and 40 MHz and ultrasonification was continued for 10 minutes. The mixture was filtered under reduced pressure and dried to obtain light yellow powdery crystal.

### Example 8

10g of benidipine hydrochloride crude crystal was dissolved by heating in 1000 mL ethanol. Ultrasonification was performed for 10 minutes at 150 W and 40 MHz. The mixture was filtered under reduced pressure and dried to obtain 2.8g crystal as light yellow powder. The mother liquor was allowed stand for 24 hours, filtered under reduced pressure and dried to obtain 4.1 g crystal as light yellow powder.

### Example 9

10g of benidipine hydrochloride crude crystal was dissolved in 100 mL ethanol + 5 mL water by heating. Ultrasonification was performed for 10 minutes at 150 W and 80 MHz. The mixture was filtered under reduced pressure and dried to obtain light yellow powdery crystal.

### Example 10

10g of benidipine hydrochloride crude crystal was dissolved in 40 mL methanol by heating. Ultrasonification was performed for 10 minutes at 200 W and 40 MHz. The mixture was filtered under reduced pressure to obtain light yellow powdery crystal.

### Example 11

10g of benidipine hydrochloride crude crystal was dissolved in 40 mL methanol + 5 mL water by heating. Ultrasonification was performed for 10 minutes at 200 W and 40 MHz. The mixture was filtered under reduced pressure to obtain light yellow powdery crystal.

### Example 12

10 g of benidipine hydrochloride crude crystal was dissolved in 30 mL dichloromethane. Ultrasonification was performed for 18 minutes at 200 W and 80 MHz. The mixture was filtered under reduced pressure to obtain light yellow powdery crystal.

### Example 13

10g of benidipine hydrochloride crude crystal was dissolved in 40 mL acetonitrile by heating. Ultrasonification was performed for 10 minutes at 150 W and 80 MHz. The mixture was filtered under reduced pressure to obtain light yellow powdery crystal.

### Example 14

10g of benidipine hydrochloride crude crystal was dissolved in 40 mL acetonitrile + 5 mL water by heating. Ultrasonification was performed for 10 minutes at 150 W and 80 MHz. The mixture was filtered under reduced pressure to obtain light yellow powdery crystal.

### Example 15

10g of benidipine hydrochloride crude crystal was dissolved in 10000 mL water by heating. Ultrasonification was performed for 20 minutes at 150 W and 80 MHz. The mixture was filtered under reduced pressure to obtain light yellow powdery crystal.

### Technical effect Example

Purity test of benidipine hydrochloride (area normalization method):
Chromatography conditions
Detector: ultraviolet absorption detector (detection wavelength: 237nm)
Chromatography column: stainless steel column: 4.6 mm x 10 cm, with octadecylsilyl (ODS) silica as filler.

Column temperature: constant, about 25°C
Mobile phase: mixed solution of 0.05 mol/L potassium dihydrophosphate solution (pH 3.0): methanol: tetrahydrofuran (65:27:8)
Flow rate: adjusted to render the retention time of benidipine hydrochloride to be about 20 min.

Chromatogram record time: about 2 times of the peak time of benidipine hydrochloride
The purity of benidipine hydrochloride obtained in Example 1 was detached to be ≥98.6%.

## Claims

1. A method for preparing highly purified benidipine hydrochloride by ultrasound technology, comprises: 1)during the pre-treatment stage including acylchloridizing the main ring of dihydropyridine and then linking the side chain to synthesize directly benidipine hydrochloride, ultrasound technology is introduced, and the reactant material is dispersed and dissolved by untrasound, and/or 2)during the post-treatment stage, ultrasound technology is introduced and crude crystal (or product obtained by heating to dryness) of benidipine hydrochloride obtained via the method described above or other possible synthetic routes is dissolved once more and then highly purified benidipine hydrochloride is obtained by untrasound.

2. The method according to Claim 1, wherein ultrasound technology is introduced into the pre-treatment stage of benidipine hydrochloride synthesis, in order to improve dispersion, dissolution and homogenization of reactants, and help to enhance the reaction speed.

3. The method according to Claim 1, wherein the crude crystal of benidipine hydrochloride may be prepared according to the method of Claim 1, but it may also be synthesized through other possible synthetic routes, for example, acylchloridizing the main ring of dihydropyridine and then joining 3-piperidinol and then benzyl; or benidipine hydrochloride may be prepared by 'one-pot' method involving3-nitrobenzaldehyde, β-aminocrotonate and acetylacetate; or benidipine hydrochloride may also be prepared by synthesis of main ring via β-aminocrotonate, or acetylacetate.

4. The method according to Claim 1, wherein the crude crystal of benidipine hydrochloride of Claim 1 is re-dissolved in suitable solvent, and then crystallization is performed for one or more times under ultrasound, and highly purified benidipine hydrochloride is obtained.

5. The method according to any one of Claims 1 and 4, wherein the crystallization under ultrasound is performed at a temperature of -78°C to 100°C, preferably -5°C to 30°C.

6. The method according to any one of Claims 1 and 4, wherein the solvent used to prepare highly purified benidipine hydrochloride is selected from the group consisting of a lower-carbon-number ketone, a lower-carbon-number alcohol, a lower-carbon-number ether, a lower-carbon-number acid, a lower-carbon-number ester, dichloromethane, chloroform, acetic anhydride or other commonly used small molecule solvents alone, or any combination of two or more solvents; preferably acetone, ethanol, methanol, N,N-dimethylformamide(DMF), acetonitrile, diethyl ether, dichloromethane and water alone, or any combination of two or more solvents.

7. The method according to any one of Claims 1 and 4, wherein the frequency of the ultrasound ranges from 10 kHz to 500 kHz, preferably 15 kHz to 100 kHz.

8. The method according to any one of Claims 1 and 4, wherein the power for the ultrasound ranges from 1 mW to 5000 W, preferably 1 W to 3000 W.

9. The method according to any one of Claims 1 and 4, wherein the duration of the ultrasound ranges from 1 minute to 24 hours, preferably 3 minutes to 120 minutes.

## Patentansprüche

1. Verfahren zur Herstellung von hochgereinigtem Benidipin-Hydrochlorid durch Ultraschalltechnologie, umfassend: 1) Einführen von Ultraschalltechnologie während der Vorbehandlungsstufe einschließlich Acylchloridieren des Hauptrings von Dihydropyridin und anschließendem Verknüpfen der Seitenkette, um BenipidinHydrochlorid direkt zu synthetisieren, und Dispergieren und Lösen des Reaktantmaterials durch Ultraschall, und/oder 2) Einführen von Ultraschalltechnologie während der Nachbehandlungsstufe und weiteres Lösen von Rohkristall (oder Produkt, das durch Erwärmen zur Trockne erhalten wurde) von Benidipin-Hydrochlorid, erhalten nach dem oben beschriebenen Verfahren oder anderen möglichen Synthesewegen, und danach Erhalten von hochgereinigtem Benipin-Hydrochlorid durch Ultraschall.

2. Verfahren nach Anspruch 1, wobei Ultraschalltechnologie in die Vorbehandlungsstufe der Synthese von Benidipin-Hydrochlorid eingeführt wird, um Dispergierung, Auflösung und Homogenisierung der Reaktanten zu verbessern und zur Steigerung der Reaktionsgeschwindigkeit beizutragen.

3. Verfahren nach Anspruch 1, wobei der Rohkristall von Benidipin-Hydrochlorid gemäß dem Verfahren von Anspruch 1 hergestellt werden kann, jedoch auch über andere mögliche Synthesewege synthetisiert werden kann, beispielsweise durch Acylchloridieren des Hauptrings von Dihydropyridin und anschließendes Anbinden von 3-Piperidinol und danach Benzyl; oder Herstellen von Benidipin-Hydrochlorid nach dem Eintopfverfahren unter Beteiligung von 3-Nitrobenzaldehyd, β-Aminocrotonat und Acetylacetat, oder auch Herstellen von BenidipinHydrochlorid durch Synthese des Hauptrings über β-Aminocrotonat oder Acetylacetat.

4. Verfahren nach Anspruch 1, wobei der Rohkristall von Benidipin-Hydrochlorid von Anspruch 1 in einem geeigneten Lösungsmittel erneut aufgelöst wird und dann einmal oder mehrfach unter Ultraschall Kristallisation durchgeführt wird und hochgereinigtes BenidipinHydrochlorid erhalten wird.

5. Verfahren nach einem der Ansprüche 1 und 4, wobei die Kristallisation unter Ultraschall bei einer Temperatur von -78°C bis 100 °C, vorzugsweise -5°C bis 30 °C durchgeführt wird.

6. Verfahren nach einem der Ansprüche 1 und 4, wobei das zur Herstellung von hochgereinigtem BenidipinHydrochlorid verwendete Lösungsmittel ausgewählt ist aus der Gruppe bestehend aus einem Keton mit niedrigerer Kohlenstoffzahl, einem Alkohol mit niedrigerer Kohlenstoffzahl, einem Ether mit niedrigerer Kohlenstoffzahl, einer Säure mit niedrigerer Kohlenstoffzahl, einem Ester mit niedrigerer Kohlenstoffzahl, Dichlormethan, Chloroform, Essigsäureanhydrid oder anderen üblicherweise verwendeten kleinmolekularen Lösungsmitteln allein oder in jeglicher Kombination von zwei oder mehreren Lösungsmitteln; vorzugsweise Aceton, Ethanol, Methanol, N,N-Dimethylformamid (DMF), Acetonitril, Diethylether, Dichlormethan und Wasser allein oder jeglicher Kombination von zwei oder mehreren Lösungsmitteln.

7. Verfahren nach einem der Ansprüche 1 und 4, wobei die Frequenz des Ultraschalls im Bereich von 10 kHz bis 500 kHz, vorzugsweise 15 kHz bis 100 kHz liegt.

8. Verfahren nach einem der Ansprüche 1 und 4, wobei die Leistung des Ultraschalls im Bereich von 1 mW bis 5000 W, vorzugsweise 1 W bis 3000 W liegt.

9. Verfahren nach einem der Ansprüche 1 und 4, wobei die Dauer des Ultraschalls im Bereich von 1 Minute bis 24 Stunden, vorzugsweise 3 Minuten bis 120 Minuten liegt.

## Revendications

1. Procédé pour la préparation de chlorhydrate de benidipine hautement purifié par la technologie des ultrasons, dans lequel : 1) pendant l'étape de prétraitement comprenant l'acylchloruration du noyau principal de la dihydropyridine et ensuite la liaison de la chaîne latérale pour synthétiser directement du chlorhydrate de benidipine, la technologie des ultrasons est introduite et la matière réactive est dispersée et dissoute par des ultrasons et/ou 2) pendant l'étape de post-traitement, la technologie des ultrasons est introduite et du cristal brut (ou du produit obtenu par chauffage jusqu'à sec) de chlorhydrate de benidipine obtenu par l'intermédiaire du procédé décrit ci-dessus ou d'autres voies de synthèse possibles est dissous une fois de plus et ensuite du chlorhydrate de benidipine hautement purifié est obtenu par des ultrasons.

2. Procédé selon la revendication 1, dans lequel la technologie des ultrasons est introduite dans l'étape de prétraitement de la synthèse de chlorhydrate de benidipine, afin d'améliorer la dispersion, la dissolution et l'homogénéisation de réactifs et d'aider à accroître la vitesse de réaction.

3. Procédé selon la revendication 1, dans lequel le cristal brut de chlorhydrate de benidipine peut être préparé selon le procédé de la revendication 1, mais il peut également être synthétisé par d'autres voies de synthèse possibles, par exemple l'acylchloruration du noyau principal de la dihydropyridine et ensuite l'adjonction de 3-pipéridinol et ensuite de benzyle ; ou du chlorhydrate de benidipine peut être préparé par un procédé « monotope » mettant en jeu du 3-nitrobenzaldéhyde, du β-aminocrotonate et de l'acétylacétate ; ou du chlorhydrate de benidipine peut également être préparé par synthèse du noyau principal par l'intermédiaire de β-aminocrotonate ou d'acétylacétate.

4. Procédé selon la revendication 1, dans lequel le cristal brut de chlorhydrate de benidipine de la revendication 1 est à nouveau dissous dans du solvant approprié et ensuite une cristallisation est effectuée une ou plusieurs fois sous des ultrasons et du chlorhydrate de benidipine hautement purifié est obtenu.

5. Procédé selon l'une quelconque des revendications 1 et 4, dans lequel la cristallisation sous des ultrasons est effectuée à une température de -78 °C à 100 °C, de préférence de -5°C à 30 °C.

6. Procédé selon l'une quelconque des revendications 1 et 4, dans lequel le solvant utilisé pour préparer du chlorhydrate de benidipine hautement purifié est choisi dans le groupe constitué par une cétone ayant un nombre inférieur d'atomes de carbone, un alcool ayant un nombre inférieur d'atomes de carbone, un éther ayant un nombre inférieur d'atomes de carbone, un acide ayant un nombre inférieur d'atomes de carbone, un ester ayant un nombre inférieur d'atomes de carbone, le dichlorométhane, le chloroforme, l'anhydride acétique ou d'autres solvants à petite molécule couramment utilisés seuls ou une quelconque association de deux ou plus de deux solvants ; de préférence l'acétone, l'éthanol, le méthanol, le *N*,*N*-diméthylformamide (DMF), l'acétonitrile, l'éther de diéthyle, le dichlorométhane et l'eau seuls ou une quelconque association de deux ou plus de deux solvants.

7. Procédé selon l'une quelconque des revendications 1 et 4, dans lequel la fréquence des ultrasons va de 10 kHz à 500 kHz, de préférence de 15 kHz à 100 kHz.

8. Procédé selon d'une quelconque des revendications 1 et 4, dans lequel la puissance pour les ultrasons va de 1 mW à 5000 W, de préférence de 1 W à 3000 W.

9. Procédé selon l'une quelconque des revendications 1 et 4, dans lequel la durée des ultrasons va de 1 minute à 24 heures, de préférence de 3 minutes à 120 minutes.
